# EUROPEAN PATENT SPECIFICATION

(11) **EP 0 565 647 B1**
(45) Date of publication and mention of the grant of the patent: **02.04.1997**
(21) Application number: 92911828.9
(22) Date of filing: 10.06.1992
(51) Int. Cl.: C12P 7/06

(54) **A METHOD OF ALCOHOLIC FERMENTATION WITH THE CATALYSTS KISSIRIS AND GAMMA-ALUMINA, AFTER THEIR REGENERATION**
ALKOHOLISCHES GÄRUNGSVERFAHREN MIT DEN REGENERIERBAREN KATALYSATOREN KISSIRIS UND GAMMA-ALUMINIUMOXID
PROCEDE DE FERMENTATION ALCOOLIQUE A L'AIDE DES CATALYSEURS KISSIRIS ET GAMMA-ALUMINE, APRES LEUR REGENERATION

(30) Priority: 08.07.1991 GR 91010304
(43) Date of publication of application: 20.10.1993
(73) Proprietor: KOUTINAS, Athanasios, GR-26500 Patras (GR); KANELLAKI, Maria, GR-26110 Patras (GR); KOUINIS, Ioannis, GR-26110 Patras (GR); KALIAFAS, Argirios, GR-26110 Patras (GR); KANA, Kyriaki, GR-26110 Patras (GR); VOLIOTIS, Stavros, GR-26110 Patras (GR); ICONOMOU, Lazaros, GR-26500 Patras (GR)
(72) Inventor: KOUTINAS, Athanasios, GR-26500 Patras (GR); KANELLAKI, Maria, GR-26110 Patras (GR); KOUINIS, Ioannis, GR-26110 Patras (GR); KALIAFAS, Argirios, GR-26110 Patras (GR); KANA, Kyriaki, GR-26110 Patras (GR); VOLIOTIS, Stavros, GR-26110 Patras (GR); ICONOMOU, Lazaros, GR-26500 Patras (GR)
(86) International application number: GR9200008
(87) International publication number: WO9301298

(56) References cited:
- JOURNAL OF FERMENTATION & BIOENGINEERING, vol. 69, no. 2, 1990, Suita (JP); T. TSOUTSAS et al., pp. 93-97/
- BIOMASS, vol. 21, no. 3, 1990, GB; M.K. HAMDY et al., pp. 189-206/
- WPI, section Ch, week 7604, Derwent Publications Ltd., London (GB); Class D, AN 76-06842X/
- BIOTECHNOLOGY & BIOENGINEERING, vol. 34, no. 1, 05 June 1989, New York, NY (US); M. KANELLAKI et al., pp. 121-125/
- APPLIED BIOCHEMISTRY & BIOTECHNOLOGY, vol. 31, no. 1, October 1991, Clifton, NJ (US); L. ICONOMOU et al., pp. 83-96/
- APPLIED BIOCHEMISTRY & BIOTECHNOLOGY, vol. 30, no. 2, August 1991, Clifton, NJ (US); A.A. KOUTINAS et al., pp. 203-216/

## Description

The invention is directed to a method of alcoholic fermentation with two catalysts , kissiris and γ-aSumina, a method which can be applied in Industry, since their catalytic action remains constant from one fermentation batch to the next. This is achieved by the regeneration of these catalysts with water treatment after a fermentation batch.

Following the energy crisis of 1973 , there appears a turn towards the production of energy from renewable sources. Such a source is the biomass , from which we may produce bioethanol through the alcoholic fermentation of sugar-rich raw materials. The alcohol may be used as an automobile fuel in a mixture with gasoline (gasohol) or may replace gasoline completely with proper modification of the engine. However , the production of fuel alcohol with the present techology faces the fact that consumption of energy in the production of alcohol is almost equal to the thermal energy produced by its combustion. Also, the production cost of alcohol in relation to that of gasoline is an additional factor wich discourages its use as an automobile fuel.

The alcohol produced worldwide with molasses as a raw material, is used as a starting material for the manufacture of alcoholic beverages (liquors, brandy, ouzo e.t.c.). It is possible, though, a part of molasses and extracts of sugar beets from which the molasses is produced,to be used as a raw material for the production of fuel alcohol following the reduction of energy consumption and of its production cost is desirable also in the production of potable alcohol which is manufactured in the factories of the existing technology.

The cost reduction can be achieved through the increase of the rate of alcoholic fermentation, while the energy cost can be reduced through the increase of the alcohol content of the fermentation product. The last has an effect on the reduction of the oil consumption in the boilers of the factory, while the rate increase of the fermentation increases the productivity and decreases the construction cost of the fermentors.

γ-alumina proved to increase significantly the alcoholic fermentation of sucrose, invert sugar and molasses while increasing the final alcohol content of the fermented liquid, in comparison to that in which no γ-alumina is added [Biotechnol.Bioeng.(1989)34(1)121-125].

Kissiris or thiraiki gi increases also significantly the rate of alcoholic fermantation of molasse [J. Ferment.Bioeng. (1990) 69(2) 93-97]. It can be used in a large number of repeated batches of fermentation with no decrease of its catalytic action, only after its regeneration. The regeneration can be done after a fermantation batch by simply washing it with water. Depending on the source of molasses, kissiris was found to show a decrease of catalytic action after three to nine batches of fermentation. However, if it is to be used in a large number of batches without replacement, something which has a direct effect on the cost, it must be regenerated. This treatment is very important for the production of alcohol from molasse with increased productivity and decreased energy consumption using this mineral. This is also true for γ-alumina, mainly in the case of molasses.

Thus, it can be stated, that kissiris as well as γ-alumina are two catalysts (or rather strengtheners of enzyme catalytic action) of alcoholic fermentation of molasses and other sugars or sugar-rich raw materials, which in order to be used, should be used in a large number of batches with the same amount of catalyst. This goal can be achieved, if the catalytic action of the catalyst remains constant from one batch to the next and for a large number of batches. This, however, can be realized through the regeneration of catalyst, the methodology of which is presented in this invention. The two catalysts show an increase of productivity in the fermentation reactors as well as an increase of the alcohol content as compared to the fermentations without kissiris and γ-alumina or with those with an unregenerated catalyst. The latter can take place also in the industry using free cells with catalyst proposed and constitute the traditional mode of fermentation in industry.

### Kissiris

The mineral kissiris is known as elaphropetra or thiraiki gi. It is a porous material and contains chiefly SiO₂, Al₂O₃ and other inorganic oxides. It is of volcanic origin and occurs in the island Thira (Santorini) and other Greek islands. In our experiments it was used after washing with water.

### A. Fermentations of molasses with kissiris in successive batches after regeneration of kissiris.

We placed in a 1-liter beaker 100 - 800g kissiris, 500 ml diluted molasses 10-17.4 °Be which contains as a nutrient phosphate salt of ammonium or of alkali metals (K, Na) with or without sterilization and baker's yeast (Saccharomyces cerevisiae) or some other yeast strain with a cell concentration greater than 10g/l (pressed yeast).

In order to compare kinetic factors in the presence of kissiris, we ran simultaneous fermentations in the absence of it using the same volume of molasses in the same initial °Be, the same amount of the same yeast and under the same, generally, conditions. The experiments were carried out at 25-32 °C with no stirring.

The pH of the molasse to be fermented was adjusted to the optimum range. When the fermentation was over, the liquid product was decanted and the kissiris was washed 3 times with 500ml water of temperature 15-100 °C or with boiling water under steam pressure, for the regeneration of the catalytic action. The catalyst was then used for the next fermentation batch which was carried out exactly as the previous one and this process was repeated many times.

A part of the results obtained are given in Table I. The method was repeated with powdered kissiris and stirring and the results were similar. The experiment was carried out also in a 3-1 feed batch reactor with pieces of kissiris, a dosometric pump and the same conditions and the results were similar.

**TABLE I.**

| Results of the method with molasses. | | | | | | |
|---|---|---|---|---|---|---|
| Ferment. batch | Initial ⁰Be | Final ⁰Be | Ferment. time | Ethanol Conc. | Ethanol Productivity | Ethanol Yield Factor |
| | density | density | (hr) | (g/l) | (g/l/24hr) | (g/g) |
| 1st | 17 | 10.8(6,4)* | 160(40) | 50(89) | 7.5(5.34) | 0.28(0.49) |
| 12th | 16.6 | 10.8(8.9) | 160(26) | - | - | - |
| 57th | 17.2 | 8.3(6.6) | 95(50) | 72(89) | 18(42.7) | 0.39(0.48) |
| 58th | 17.3 | 8.3(6.7) | 95(49) | 68(83) | 17(40.7) | 0.37(0.45) |

| | | | | | | |
|---|---|---|---|---|---|---|
| * Values in parentheses are those obtained in the presence of kissiris. | | | | | | |

### γ-Alumina.

Pellets of γ-alumina Houndry Ho 415 with a porosity 0.45 cm³ /g and specific surface area 1.40m² /g. γ-Alumina was also employed as powder after sifting it through sieves.

### B. Fermentations of molasses with γ-alumina pellets in successive batches.

We placed in a 1-liter beaker 70-1000 g of γ-alumina pellets, 500 ml diluted molasses 10-17.4 °Be which contains as nutrient phosphate salt of ammonium or of alkali metals (K, Na) with or without sterilization and baker's yeast (Saccharomyces cerevisiae) or some other strain of yeast with a cell concentration greater than 10 g/l (pressed yeast).

In order to compare kinetic factors in the presence of γ-alumina we carried out simultaneous fermentantions in the absence of γ-alumina, using the same volume of molasse, in the same initial ^{°}Be and with the same amount of the same yeast and under the same, generally, conditions. The experiments were performed at 25-32 °C and no stirring.

The pH of the molasses to be fermented was adjusted to the optimum range. The pH was adjusted also during the fermentation to the optimum range. When the fermentation was completed, the liquid product was decanted and the alumina was washed 3 times with water of temperature 5-100 °C or with boiling water under steam pressure, for the regeneration of the catalytic action. The catalyst was used then for the next fermentantion batch, carried out exactly as the previous one, and the process was repeated many times.

A part of the results obtained are presented in Table II. The experiment was repeated in a 3-1 feed batch reactor employing a dosometric pump and the same conditions and gave similar results.

**TABLE II.**

| Ferment. batch | Initial conc. of sugar | Ferment. time | Ethanol conc. | Ethanol Productivity | Residual sugar | Conversion | Ethanol Yield Factor |
|---|---|---|---|---|---|---|---|
| | (g/l) | (hr) | (g/l) | (g/l/24hr) | (g/l) | (%) | (g/l) |
| 1st | 179.5 | 144(57) | 53.9(78.4) | 9.0(33.0) | 66.9(19.0) | 62.7(89.4) | 0.30(0.44) |
| 8th | 193.0 | 120(65) | 58.4(83.2) | 11.7(30.7) | 71.0(19.4) | 64 (90) | 0.30(0.43) |
| 13th | 193.0 | 120(47) | 58.4(83.2) | 11.7(42.4) | 71.0(15.7) | 64 (92) | 0.30(0.43) |
| 18th | 196.5 | 120(70) | 58.4(84.8) | 11.7(29.0) | 71.0(16.6) | 64 (91) | 0.30(0.43) |
| The values in parentheses are those obtained in the presence of γ-alumina. | | | | | | | |

Since the molasses is a product of sugar beets and sugar canes, the effect should be analogous for their extracts.

### C. Fermentations of sucrose, fructose and invert sugar solutions.

In a 1-liter beaker were placed 70-1000 g of γ-alumina pellets, 500 ml of sucrose, fructose or invert sugar solutions, 215 g/l containing lg/l (NH₄)₂SO₄ , 5 g/l MgSO₄ and 4 g/l of yeast extract. To this mixture were added baker's yeast (Sacharomyces cerevisiae) or some other strains of yeast with a cell concentration greater than 10 g/l (pressed yeast). In order to compare the kinetic factors of fermentations in the presence of γ-alumina, fermentations were carried out with the same volume of sugar solution, same concentrations of nutrients, same amount of the same yeast and the same, generally conditions, with no γ-alumina pellets. The experiments were performed at 20-32 °C with no stirring. The pH was adjusted to the optimum range for sucrose and to 3.2-6.5 for invert sugar and fructose. A part of the results obtained appear in Table III.

**TABLE III**

| Sugar | Initial sugar conc. | Ferment. time | Residual sugar | Ethanol Yield Factor |
|---|---|---|---|---|
| | (g/l) | (hr) | (g/l) | (g/g) |
| Fructose | 215 | 24(10)* | 29(17) | 0.41(0.44) |
| Sucrose | 215 | 25(16) | 52(10) | 0.36(0.46) |
| Invert Sugar | 215 | 24(14) | 48(12) | 0.37(0.45) |

| | | | | |
|---|---|---|---|---|
| * The values in parentheses are those obtained in the presence of γ-alumina. | | | | |

### D. Repeated fermentations of glucose solutions with γ-alumina pellets, following the regeneration of catalytic action.

In a 1-liter beaker were placed 70-1000 g of γ-alumina pellets and 500 ml of 215 g/l glucose solution containing 1 g/l KH₂PO₄, 1 g/l (NH₄)₂SO₄, 5 g/l MgSO₄ and 4 g/l of yeast extact.

To the mixture was added baker's yeast (Saccharomyces cerevisiae) or some other strains of yeast in cell concentration greater than 10 g/l (as pressed yeast).

In order to compare the kinetic factors of fermentation in the presence of γ-alumina, fermentations were carried out in the absence of γ-alumina in the same volume of glucose solutions with the same concentrations of nutrients the same amount of the same yeast and the same, generally conditions. The experments were run at 20-32 °C with no stirring and the pH adjusted to 3.2-6.5.

At the end of the fermentation the liquid was decanted and the pellets were used in another fermentation batch with or without regeneration if this is not required. The regeneration is accomplished with water of temperature 15-100 °C or with boiling water understeam pressure. Thus, a large number of fermentation batches were carried out with the same amount of γ-alumina.

The experiment was carried out in a 3-1 feed batch reactors, employing a dosometric pump and the same conditions and gave similar results.

### E. Fermentations of glucose with γ-alumina powder.

In a 1-liter beaker containing 70-300 g of powdered alumina were added 500 ml of 215 g/l glucose solution containing 1 g/l KH₂PO₄ , 1 g/l (NH₄)₂SO₄ , 5 g/l MgSO₄ and 4 g/l of yeast extact.

To the mixture was added baker's yeast (Saccharomyces cerevisiae) or some other strains of yeast in cell concentration greater than 10 g/l (as pressed yeast). During the fermentation the mixture was stirred with a magnetic stirrer. In order to compare the kinetic factors of fermentation in the presence of γ-aluminum powder, fermentations were performed without γ-alumina, in the same volume of glucose solutions, with the same concentrations of nutrients, the same amount of the same ueast and under the same, generally, conditions. The pH was adjusted to 3.2-6.5 and the temperature to 20-32 °C. The results obtained are shown in Table IV.

**TABLE IV.**

| Particle size of γ-alumina (µm) | Initial sugar conc. (g/l) | Ferment. time (hr) | Residual sugar (g/l) | Ethanol Yield Factor (g/g) |
|---|---|---|---|---|
| No γ-Al2O3 | 215 | 70 | 20 | 0.42 |
| pellets | 215 | 32 | 16 | 0.43 |
| 850 | 215 | 24 | 2 | 0.48 |
| 212 | 215 | 29 | 15 | 0.43 |

## Claims

1. A method of alcoholic fermentation with the use of kissiris and γ-alumina, characterized by the fact that the fermentation of molasses, sucrose, invert sugar, glucose, juices or extracts of raw materials can be promoted, by the presence of each one of the above inorganic materials separately and after regeneration of the kissiris and gamma-alumina from batch to batch.

2. A method of alcoholic fermentation, as stated in the claim 1, characterized by the fact that the two catalysts can be used for a large number of succesive batches,after their regeneration with water of temperature 15-100°C.

3. A method of alcoholic fermentation, as stated in the claim 1, characterized by the fact that the two catalysts can be used for a large number of succesive batches, after their regeneration with boiling water under steam pressure.

4. A method of alcoholic fermentation, as stated in the claim 1, characterized by the fact that the γ-alumina in the fermentation of molasses can be used also in the powder form, to improve results obtained with γ- alumina pellets.

5. A method of alcoholic fermentation, in accordance with the claims 1, 2, 3, and 4 characterized by the fact that the fermentation with glucose can be accomplished with the stirring of γ-alumina powder.

6. A method of alcoholic fermentation, in accordance with the claims 1, 2 and 3, characterized by the fact that the kissiris in the molasses fermentation can be used in powder form.

7. A method of alcoholic fermentation, in accordance with the claims 1, 2, 3, 4, 5 and 6 characterized by the fact that the fermentation can be accomplished with any species of yeast which affects an alcoholic fermentation.

8. A method of alcoholic fermentation, in the molasse and glucose fermentations in succesive batches as related with claims 4,5,6, and 7 in accordance with the claims 1,2,3, characterised by the fact that the fermentation can be accoplished in a feed batch reactor.

9. A method of alcoholic fermentation, in accordance with the claims 1, 2, 3, 4, 5, 6, 7, and 8 characterised by the fact that there can be proportions of weight of γ-alumina or kissiris to the volume of the liquid in the range of 70-1000g/500 ml.

10. A method of alcoholic fermentation of molasses, as is stated in the claims 1, 2, 3, 4, 5, 6, 7, 8, 9, characterized by the fact that it can be fermented in 10-17.4 °Be and contains one of the phospate salts of ammonium or one of the corresponding to the alkali metals (K, Na).

11. A method of alcoholic fermentation,as it is stated in the claims 1,2, 3, 4, 5, 6,7,8,9 and 10, characterized by the fact that there can be any concentration of yeast greater than 10gr/1(as pressed yeast)or of any species of yeast which can affect an alcoholic fermentation.

12. A method of alcoholic fermentation, in accordance with the claims 1,2,3,4,5,6,7,8,9,10 and 11,
characterized by the fact that there can be any temperature of fermentation within the range of 20-32 °C.

13. A method of alcoholic fermentation,in accordance with the claims 1,2,3,4,5,6,7,8,9,10,11 and 12, characterised by the fact that the fermentation,can be achieved in any pH value, within the range of 3.2-6.5.

## Patentansprüche

1. Ein Verfahren der alkoholischen Gärung unter Verwendung von Kissiris und γ-Alumina, bei der in Gegenwart jedes einzelnen der eben genannten anorganischen Stoffe, und nach Regeneration des Kissiris und des γ-Aluminas von einer Reaktorfüllung zu Nächsten, die Gärung von Molasse, Sukrose, Inverszucker, Glukose, Säften oder Extrakten von Rohstoffen verbessert werden kann.

2. Ein Verfahren der alkoholischen Gärung, wie in Anspruch 1 genannt, insofern, als die beiden Katalysatoren wiederholt in einer hohen Anzahl von Reaktorfüllungen genutzt werden können, nachdem sie mit Wasser bei Temperaturen zwischen 15-100°C regeneriert wurden.

3. Ein Verfahren der alkoholischen Gärung, wie in Anspruch 1 genannt, insofern, als die beiden Katalysatoren wiederholt in einer hohen Anzahl von Reaktorfüllungen genutzt werden können, nachdem sie mit siedendem Wasser unter Dampfdruck regeneriert wurden.

4. Ein Verfahren der alkoholischen Gärung, wie in Anspruch 1 genannt, insofern, als das γ-Alumina bei der Gärung von Molasse auch in Pulverform eingesetzt werden kann um die mit γ-Alumina-Pellets erzielten Ergebnisse zu verbessern.

5. Übereinstimmung mit den Ansprüchen lmit 4, insofern, als die Glucosegärung mittels Rühren von γ-Alumina-Pulver ausgefürt werden kann

6. Ein Verfahren der alkoholischen Gärung , in Übereinstimmung mit den Ansprüchen lmit 3, insofern, als bei der Gärung von Molasse Kissiris in Pulverform verwendet werden kann.

7. Ein Verfahren der alkoholischen Gärung , in Übereinstimmung mit den Ansprüchen lmit 6, insofern, als die Gärung mit jeder Hefeart, die alkoholisch zu Gären vermag, verwirklicht werden kann.

8. Ein Verfahren der alkoholischen Gärung, bezogen auf die Ansprüche 4 mit 7 und in Übereinstimmung mit den Ansprüchen lmit 3, insofern, als Molasse und Glukose in wiederholten Reaktorfüllungen, in zugefütterten Reaktoren vergärt werden.

9. Ein Verfahren der alkoholischen Gärung, in Übereinstimmung mit den Ansprüchen lmit 8, insofern, als die Verhältnisse der Massen des γ-Aluminas oder Kissiris zum Volumen der Lösung in Größenordnungen von 10-1000 g/500ml stehen.

10. Ein Verfahren der alkoholischen Gärung, wie in den Ansprüchen 1 mit 9 beschrieben, insofern, als sie bei 10-17,4 °Be abläuft und ein Phosphat des Ammoniums oder der korrespondierenden Alkalimetalle (K,Na) enthält.

11. Ein Verfahren der alkoholischen Gärung, wie in den Ansprüchen 1 mit 10 beschrieben, insofern, als die Konzentration der Hefe jeden Wert größer als 10 g/L annehmen kann, als gepreßte Hefe oder in Form jeder anderen Hefeart.

12. Ein Verfahren der alkoholischen Gärung, in Übereinstimmung mit den Ansprüchen 1 mit 11, insofern, als die Temperatur während der Gärung jeden Wert zwischen 20-32° annehmen kann.

13. Ein Verfahren der alkoholischen Gärung, in Übereinstimmung mit den Ansprüchen 1 mit 12, insofern, als die Gärung bei jedem pH-Wert zwischen 3.2 - 6.5 ausgeführt werden kann.

## Revendications

1. Une méthode de fermentation alcoolique avec l'emploi de kissiris et χ-alumina, caractérisée par le fait que la fermentation de mélasse, sucrose, sucre inversé, glucose, jus ou extraits de matière première peuvent être promûs, par la présence de chacun une des matières au-dessus inorganiques isolément et après la régénération du kissiris et γ- alumine de fournée à fournée.

2. Une méthode de fermentation alcoolique, comme énonçait dans la revendication 1, caractérisée par le fait que les deux catalyseurs peuvent être employés pour un grand nombre de fournées successives, après leur régénération avec de l'eau de température 15-100 °C.

3. Une méthode de fermentation alcoolique, comme énonçait dans la revendication 1, caractérisée par le fait que les deux catalyseurs peuvent être utilisés pour un grand nombre de fournées successives, après leur régénération avec de l'eau bouillantdessous à la pression de vapeur.

4. Une méthode de fermentation alcoolique, comme énonçait dans la revendication 1, caractérisée par le fait que le γ-alumine dans la fermentation de mélasse peut être utilisé aussi dans la forme de poudre, à améliorer des résultats obtenue avec γ-alumine boulettes.

5. Une méthode de fermentation alcoolique, conformément aux revendications 1, 2, 3 et 4 caractérisée par le fait que la fermentation de glucose accomplie avec le mélange de γ-alumine.

6. Une méthode de fermentation alcoolique, conformément aux revendications 1, 2 et 3, caractérisée par le fait que le kissiris dans la fermentation de mélasse utilisé dans la forme de poudre.

7. Une méthode de fermentation alcoolique, conformément aux revendications 1, 2, 3, 4, 5 et 6 caractérisée par le fait que la fermentation peut être accomplie avec toute espèce de levure qui affecte une fermentation alcoolique.

8. Une méthode de fermentation alcoolique, dans les fermentations de glucose et mélasse dans des fournées successives comme se liés avec des revendications 4, 5, 6 et 7 conformément aux revendications 1, 2, 3, caractérisée par le fait que la fermentation peut être accomplie dans un réacteur de fournée de nourrir.

9. Une méthode de fermentation alcoolique, conformément aux revendications 1, 2, 3, 4, 5, 6, 7 et 8 caractérisée par le fait qu'il peut y avoir des proportions de poids de γ-alumine ou kissiris au volume du liquide dans la gamme de 10-1000/500ml.

10. Une méthode de fermentation alcoolique de mélasse, comme est énoncée dans les revendications 1, 2, 3, 4, 5, 6, 7, 8, 9, caractérisée par le fait qu'il peut être fermenté dans 10-17,4 °Be et contient un des sels de phosphate d' ammonium ou un qui correspondre aux métaux d'alcali (K, Na).

11. Une méthode de fermentation alcoolique, comme ell' est énoncé dans les revendications 1, 2, 3, 4, 5, 6, 7, 8, 9 et 10, caractérisée par le fait qu'il peut y avoir toute concentration de levure plus grande que 10 gr / l (comme la levure pressée) ou de toute espèce de levure qui peut affecter une fermentation alcoolique.

12. Une méthode de fermentation alcoolique, conformément aux revendications 1, 2, 3, 4, 5, 6, 7, 8, 9, 10 et 11, caractérisée par le fait qu'il peut y avoir toute température de fermentation dans la gamme de 20 - 32 °C.

13. Une méthode de fermentation alcoolique, conformément aux revendications 1, 2, 3, 4, 5, 6, 7, 8, 9, 10, 11 et 12, caractérisée par le fait que la fermentation peut être réalisée dans toute pH valeur, dans la gamme de 3,2-6,5.
